# EUROPEAN PATENT APPLICATION

(11) **EP 1 306 066 A1**
(43) Date of publication of application: **02.05.2003**
(21) Application number: 01125127.9
(22) Date of filing: 23.10.2001
(51) Int. Cl.: A61F 5/453, A61F 13/15

(54) **Urine bag for male user**

(71) Applicant: Chen, Tsai-Yuan, Keelung-city (TW)
(72) Inventor: Chen, Tsai-Yuan, Keelung-city (TW)
(74) Representative: Kihn, Pierre Emile Joseph

(57) **Abstract**

A urine bag for a male user has a pouch (10) with an opening (11) defined in the pouch (10), a fastener (20) mounted around a periphery defining the opening (11), and an arcuate piece (30) mounted between the arcuate piece (30) and the periphery. The pouch (10) is composed of a permeable layer (12), an absorbent layer (14), and a waterproof layer (16). The urine bag can be worn to collect urine from a user who does not have convenient access to a toilet, and the urine bag is disposable after use.

## Description

### 1. Field of the Invention

The present invention relates to a urine bag, and more particularly to a urine bag for a male user. The urine bag can be worn to collect urine from a user who does not have convenient access to a toilet.

### 2. Description of Related Art

There are certain occasions when a person may not have a convenient access to a toilet and the most common occasion is when driving on a freeway. When driving on a freeway especially in a traffic jam, urination is troublesome because it may not be easy to find a toilet within a reasonable time. Some people would pull over and look for a shelter to urine, but this solution is embarrassing and not hygienic. Although an adult diaper may be a solution for urination when a toilet can not be easily accessed, wearing the adult diaper is inconvenient and embarrassing. Moreover, the adult diaper can not be conveniently taken off after use, and to wear the adult diaper for a period of time is a burden to a wearer.

Accordingly, the present invention tends to provide a urine bag for a male user to mitigate or obviate the aforementioned problems.

An objective of the present invention is to provide a urine bag for a male user that can solve the urination problem of a male user when a toilet can not be conveniently accessed.

Another objective of the present invention is to provide a urine bag for a male user that can be conveniently worn and taken off.

Another objective of the present invention is to provide a urine bag for a male user that is disposable after use.

Other objectives, advantages and novel features of the invention will become more apparent from the following detailed description when taken in conjunction with the accompanying drawings.

In the drawings:
Fig. 1 is a perspective view of a urine bag for a male user in accordance with the present invention;
Fig. 2 is an enlarged, perspective view showing a fastener of the urine bag; and
Fig. 3 is a cross-sectional view of the urine bag in use.

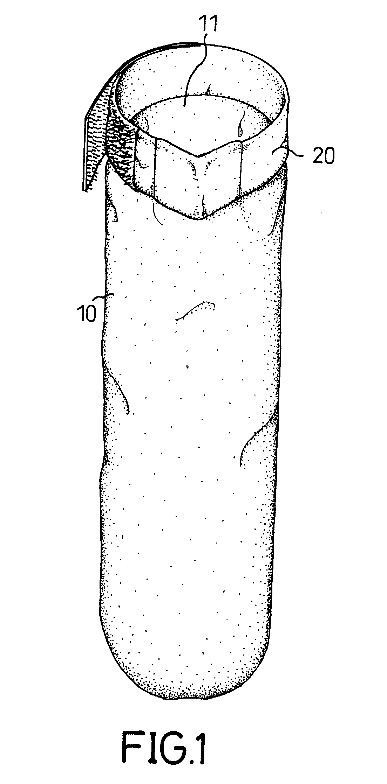

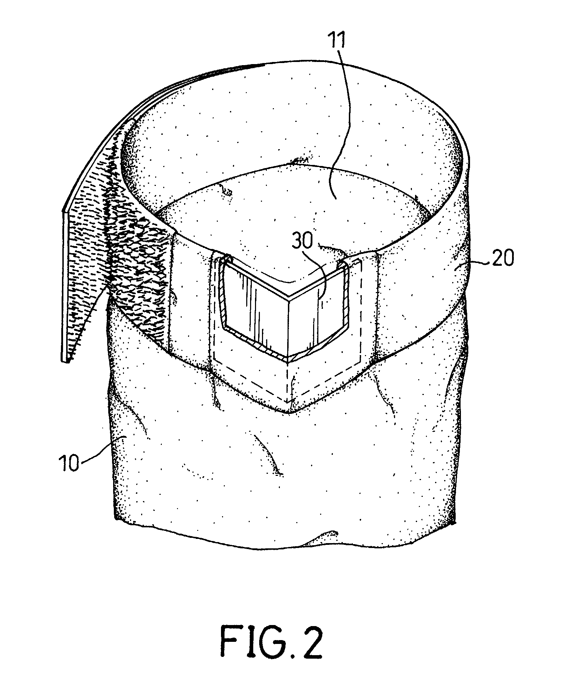

With reference to Figs. 1 and 2, a urine bag for a male user in accordance with the present invention has a pouch (10), a fastener (20), and an arcuate piece (30).

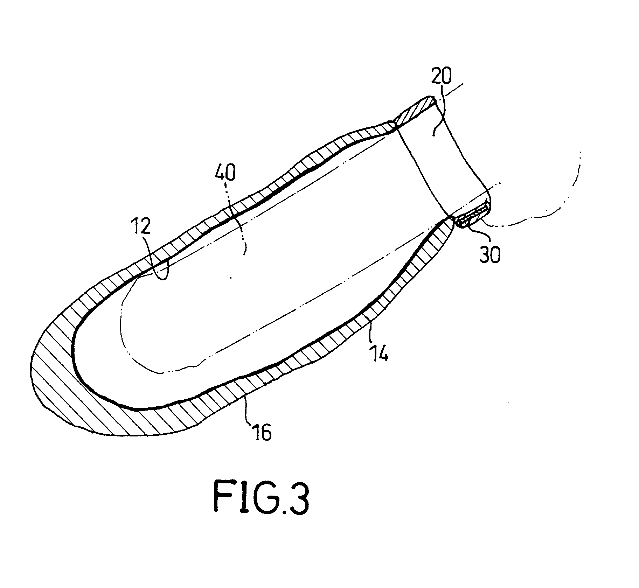

With reference to Figs. 1, 2 and 3, the pouch (10) has the size and shape corresponding to a human penis (40). The pouch (10) has an opening (11) defined therein and the pouch (10) is composed of a permeable layer (12), an absorbent layer (14), and a waterproof layer (16). The permeable layer (12) forms an inner layer of the pouch (10). The waterproof layer (16) forms an outer layer of the pouch (10) to seal the urine inside the pouch (10). The absorbent layer (14) is a layer between the permeable layer (12) and the waterproof layer (16). The absorbent layer (14) is made of a material with high absorbability.

The fastener (20) is mounted around a periphery defining the opening (11) of the pouch (10). The fastener (20) can be of various kinds and a hook and loop fastener as shown in the figures is a preferred kind.

The arcuate piece (30) is securely mounted between the fastener (20) and the periphery defining the opening (11) to reserve a space for a male urethra.

When in use, a user places his penis (40) into the pouch (10) through the opening (11) and the fastener (20) is fastened to seal the opening (11). The arcuate piece (30) is arranged at a bottom side of the penis (50). Then the user can urinate at will and the urine permeates through the permeable layer (12) and is absorbed by the absorbent layer (14). However, the waterproof layer (16) outside the absorbent layer (14) prevents the urine from further permeating out of the pouch (10). It is noted that the male urethra is near the bottom side of the penis (40), and the arcuate piece (30) reserves a space such that when the user fastens the fastener (20), the male urethra will not be constricted by the fastener (20), thereby allowing comfortable urination. After the urination, the user can withdraw his penis and further fasten the fastener (20) to seal the urine inside the urine bag. The urine bag is designed for one time use and is disposable afterwards.

From the above description, it is noted that the invention has the following advantages:
1. The urine bag can solve the urination problem for male users during driving or other occasions when a toilet can not be conveniently accessed.
2. The urine bag can be conveniently worn and taken off.
3. The urine bag has an arcuate piece (30) for unrestricted urination.
4. The urine bag is disposable.

## Claims

1. A urine bag for a male user comprising:
a pouch (10) having:
a permeable layer (12) formed in an inner layer of the pouch (10);
a waterproof layer (16) formed in an outer layer of the pouch (10); and
an absorbent layer (14) formed between the permeable layer (12) and the waterproof layer (14);
an opening (11) defined in the pouch;
a fastener (20) mounted around a periphery defining the opening (11) to fasten the opening (11); and
an arcuate piece (30) mounted between the fastener (20) and a periphery of the pouch (10) defining the opening (11) to reserve a space such that a male urethra of a user will not be constricted when the fastener (20) is fastened,
whereby the user is able to urinate in the pouch.

2. The urine bag as claimed in claim 1, wherein the fastener (20) is a hook and loop fastener.
